# EUROPÄISCHE PATENTSCHRIFT

(11) **EP 0 640 358 B1**
(45) Veröffentlichungstag und Bekanntmachung des Hinweises auf die Patenterteilung: **27.01.1999**
(21) Anmeldenummer: 94112858.9
(22) Anmeldetag: 18.08.1994
(51) Int. Cl.: A61M 39/10, A61M 39/12

(54) **Schlauchkupplung**
Tube coupling
Raccord de tuyaux

(30) Priorität: 24.08.1993 DE 4328409
(43) Veröffentlichungstag der Anmeldung: 01.03.1995
(60) Teilanmeldung: 98111600.7
(73) Patentinhaber: B. BRAUN MELSUNGEN AG, 34212 Melsungen (DE)
(72) Erfinder: Eick, Rüdiger, D-66459 Kirkel (DE); Harms, Heidemarie, D-45143 Essen (DE); Mehner, Gotthilf, D-66280 Sulzbach (DE)
(74) Vertreter: Selting, Günther, Dipl.-Ing.

(56) Entgegenhaltungen:
- WO-A-81/00053
- DE-A- 2 907 832
- US-A- 4 201 406
- US-A- 4 433 973
- US-A- 4 673 400

## Beschreibung

### Technisches Gebiet

Die Erfindung bezieht sich auf eine Schlauchkupplung zum lösbaren Verbinden eines von einer Saugvorrichtung kommenden Schlauches mit einem unlösbar mit der Schlauchkupplung verbundenen Drainageschlauch.

### Stand der Technik

Bei der medizinischen Unterdruckdrainage besteht das Problem, den z.B. aus einer Operationswunde herausführenden Drainageschlauch lösbar mit einer Saugvorrichtung, insbesondere einer evakuierten Saugflasche (Redon-Flasche) zu verbinden. Es werden hierzu verschiedene Steckverbindungen benutzt. Bei einer bekannten Schlauchkupplung ist der Drainageschlauch fest mit einer Schlauchkupplung verbunden. Der von der Saugeinrichtung kommende Schlauch wird in einen ringspaltförmigen Aufnahmeraum eingeführt, der durch die Außenseite eines rohrförmigen Stutzens und die Innenseite eines rohrförmigen Außengehäuses gebildet wird. Nachteilig an diesem Verbinder ist die Schwierigkeit, den von der Saugeinrichtung kommenden Schlauch in den ringspaltförmigen Aufnahmeraum einzubringen und dabei auf den rohrförmigen Stutzen aufzustecken. Um die Verbindung wieder lösen zu können sind erhebliche Kräfte erforderlich, wobei die Trennung plötzlich und ruckartig erfolgt. Hierdurch wird ein in den Schläuchen noch bestehendes Vakuum sehr plötzlich belüftet, was zum Verspritzen von möglicherweise kontaminiertem Sekret führen kann.

Aus der US-A-4 201 406 ist eine Schlauchkupplung mit einem unlösbar mit ihr verbundenen Schlauch bekannt. Diese Schlauchkupplung weist am freien Ende einen zylindrischen Stutzen mit einer Abschrägung auf, der zusammen mit einem Außengehäuse einen Schlauchaufnahmeraum bildet.

### Beschreibung der Erfindung

Aufgabe der vorliegenden Erfindung ist die Zurverfügungstellung einer verbesserten Schlauchkupplung, die ein einfaches und sicheres Verbinden von Saug- und Drainageschlauch und ein Trennen der beiden Schläuche unter verlangsamter Aufhebung eines noch bestehenden Unterdrucks erlaubt.

Erfindungsgemäß gelöst wird diese Aufgabe dadurch, daß bekannte Schlauchkupplungen hinsichtlich des durch einen Stutzen und ein Außengehäuse gebildeten Schlauchaufnahmeraumes abgewandelt werden, indem der Stutzen und die Innenseite des Außengehäuses konisch gestaltet sind und der Stutzen auf seiner Außenseite etwa ab der Mitte mindestens eine in patientenferner Richtung bis zum patientennahen Ende der Abschrägung laufende Nut aufweist.

Gegenstand der Erfindung ist daher eine Schlauchkupplung zum lösbaren Verbinden eines von einer Saugvorrichtung kommenden Schlauches mit einem unlösbar mit der Schlauchkupplung verbundenen Drainageschlauch und mit einem auf der patientenfernen Seite angeordneten Stutzen, der an einem Ende eine Abschrägung aufweist und zusammen mit einem Außengehäuse einen in patientenferner Richtung offenen Schlauchaufnahmeraum bildet, dadurch gekennzeichnet, daß der Stutzen und die Innenseite des Außengehäuses konisch gestaltet sind und der Stutzen auf seiner Außenseite etwa ab der Mitte mindestens eine in patientenferner Richtung bis zum patientennahen Ende der Abschrägung laufende Nut aufweist.

In einer bevorzugten Ausführungsform weist der Stutzen in seinem patientennahen Bereich mindestens eine seitliche Abflachung auf. Bevorzugt weist der Stutzen regelmäßig auf seinem Umfang verteilte Abflachungen auf.

Die erfindungsgegenständliche Schlauchkupplung wird nach den dem Fachmann geläufigen Verfahren aus den in der Medizintechnik für solche Gegenstände üblichen Materialien hergestellt.

Nachstehend wird die Erfindung anhand eines Ausführungsbeispieles näher beschrieben.

### Es zeigen:

- Fig. 1a - 1d: verschiedene Ausführungen der Schlauchkupplung in etwa fünffacher Vergrößerung
- Fig. 2a-2d: Querschnitte in den Ebenen 11-11 der Fig. 1a bis Fig. 1d
- Fig. 3: einen Querschnitt in der Ebene 111-111 der Fig. 1c
- Fig. 4: einen Längsschnitt durch die Schlauchkupplung in Funktionsstellung, d.h. zwei verschiedene Schläuche kuppelnd, in etwa zweifacher Vergrößerung
- Fig. 5: eine Darstellung entsprechend der Fig. 3, jedoch den Zustand des Entkuppelns darstellend.

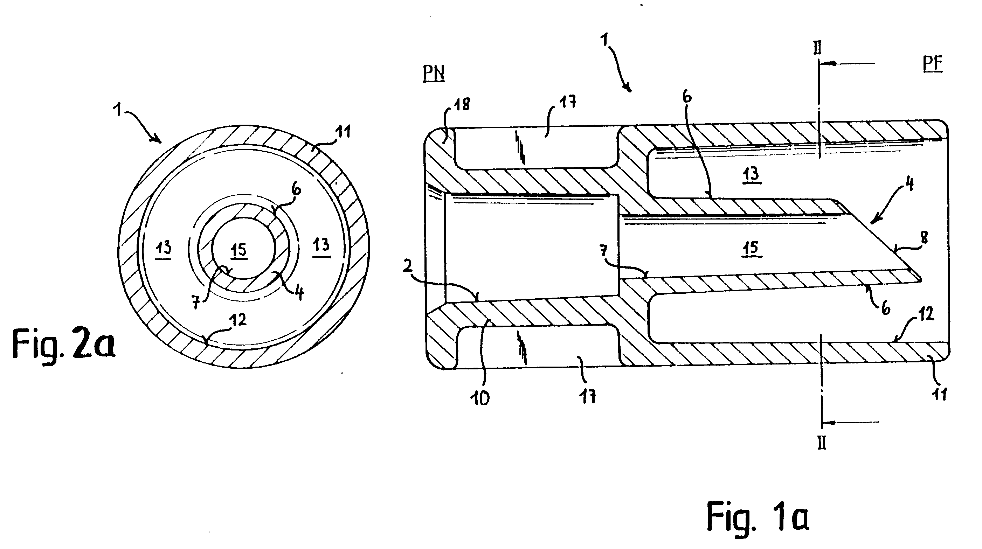

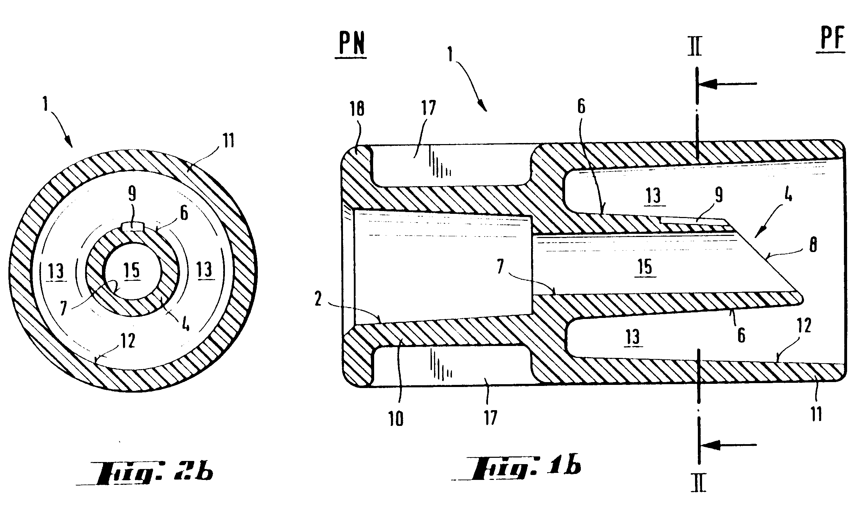

(Bei den in Fig. 1a, 1c, 2a, 2c und 3 gezeigten Schlauchkupplungen handelt es sich nicht um Ausführungsbeispiele der Erfindung.)

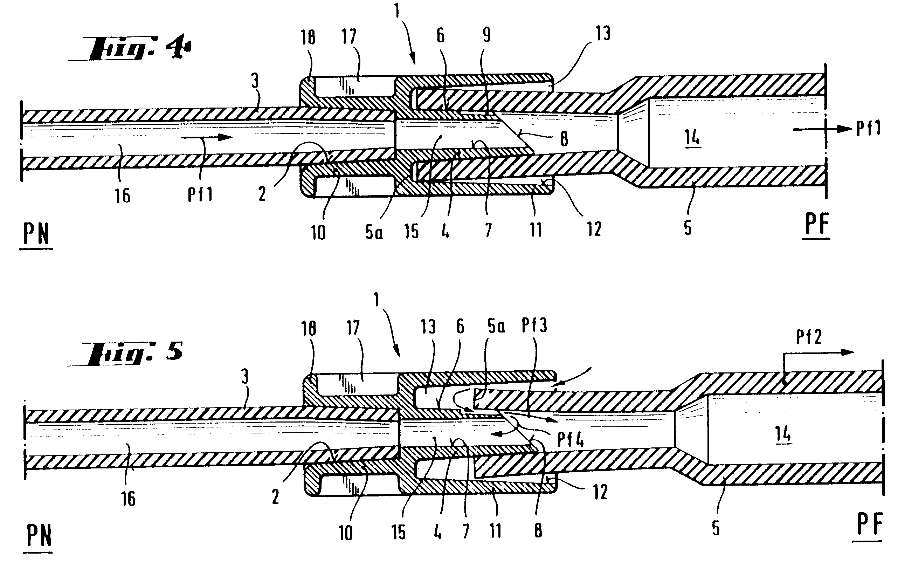

Die Schlauchkupplung 1 weist auf ihrer patientennahen Seite PN einen Stutzen 10 mit konischer Bohrung 2 zur vorzugsweise unlösbaren Aufnahme eines Saugschlauches 3 auf. Auf seiner patientenfernen Seite PF ist ein Anschlußstutzen 4 zur Aufnahme eines Saugschlauches 5 (Fig. 4, vorgesehen, welcher zu einem nicht dargestellten Auffanggefäß führt.

Der Stutzen 4 ist außen mit einem Konus 6 versehen und ist innen von einer Bohrung 7 durchsetzt, weiche das Durchtritts-Lumen 15 für die abzusaugende Körperflüssigkeit bildet. Zur Erleichterung der Applikation des Schlauches 5 auf den Konus 6, bei welchem sich das Ende des Schlauches 5 aufweitet, endet Stutzen 4 in einer Abschrägung 8.

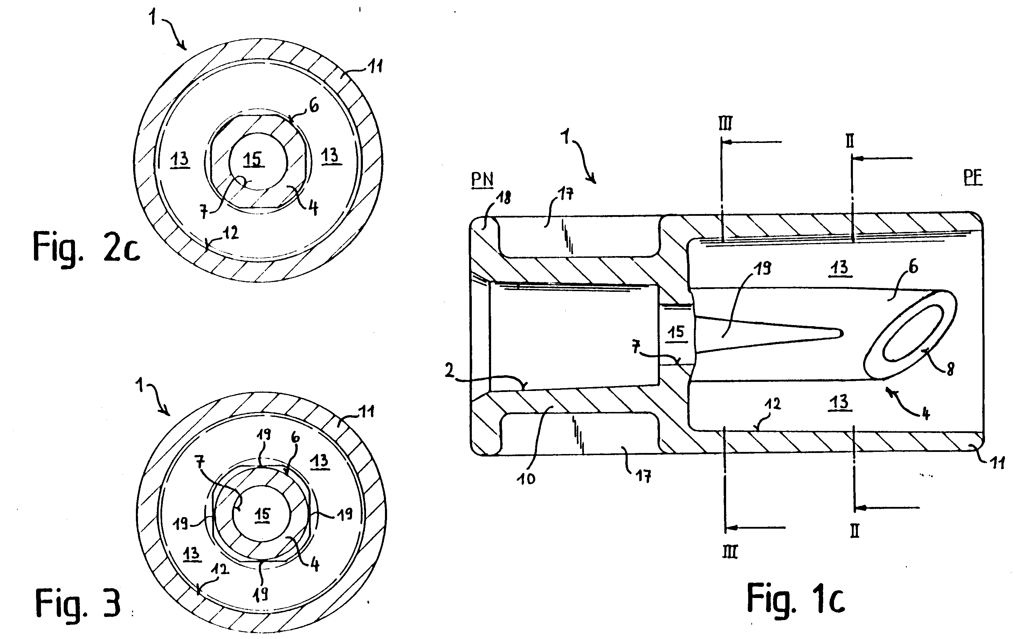

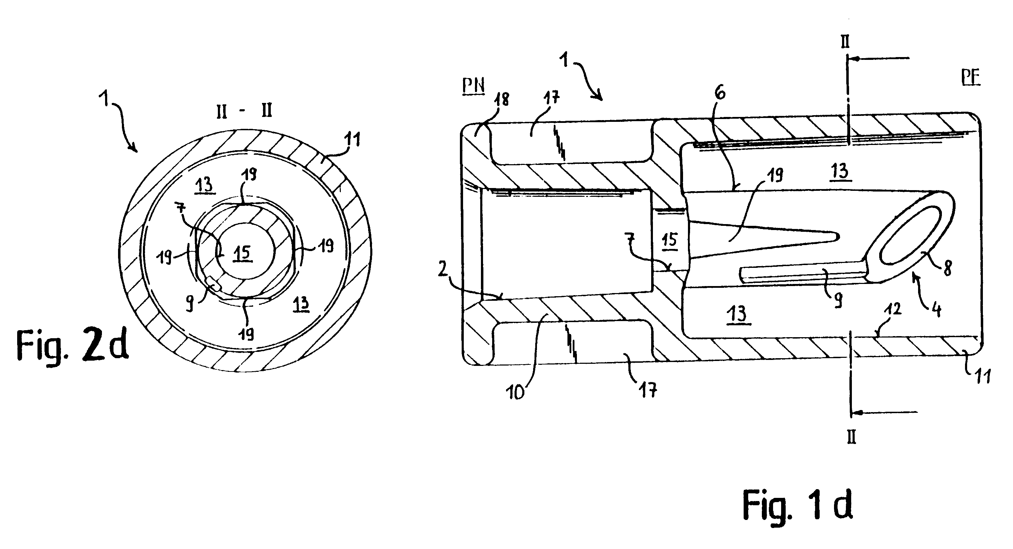

Erfindungsgemäß ist im Bereich des Konus 6 mindestens an einer Stelle eine an der Abschrägung 8 endende Nut 9 vorgesehen (Fig. 1 b, 4, 5).

In einer besonderen Ausführungsform weist der Stutzen in seinem patientennahen Bereich seitliche Abflachungen 19 auf (Fig. 1c, 2c). Diese seitlichen Abflachungen 19 sind vorzugsweise gleichmäßig über den Umfang des Stutzens verteilt, so daß sich ein Querschnitt ergibt, der dem einer Schraubenmutter ähnelt (Fig. 2c, 2d, 3).

Zweckmäßigerweise können die Nut 9 bzw. die Nuten 9 zwischen benachbarten Abflachungen angebracht sein.

Ferner weist die Schlauchkupplung 1 ein Außengehäuse 11 auf, weiches auf seiner Innenseite 12 konisch ausgebildet ist. Somit entsteht durch das Außengehäuse 11 mit Innenkonus 12 und Stutzen 4 mit Außenkonus 6 ein in Richtung Patientenferne PF sich erweiternder Schlauchaufnahmeraum 13. Durch diese Erweiterung läßt sich der Schlauch 5 leicht in die Kupplung 1 einstecken, wie in Fig. 4 ersichtlich. Zu erkennen ist, daß sich hierbei das Schlauchende 5a des Schlauches 5 in Anpassung an die Konizität 6 des Stutzens 4 erweitert und damit eng und dichtend auf den Konus 6 des Stutzens 4 aufgeschoben ist.

Die Schlauchkupplung 1 und die beiden Schläuche 3 und 5 arbeiten nun dermaßen, als daß aus dem Körper des Patienten bzw. aus dessen Wunde Flüssigkeit in Richtung der Pfeile Pf 1 abgesaugt wird.

Soll nun, wie Fig. 4 zeigt, die Schlauchverbindung 3, 5 getrennt werden, so wird in Richtung des Pfeiles Pf 2 der patientenferne Schlauch 5 zweckmäßigerweise unter axialem Verdrehen der Schlauchkupplung 1 gegenüber Schlauch 5 (Pf 2) aus der Schlauchkupplung 1 gezogen. Dabei gleitet das Ende 5a des Schlauches 5 in Richtung PF am Konus 6 entlang. Zu Beginn des Abziehens des Schlauches 5 ist bedingt durch die dabei auftretende Schlauchkontraktion eine relativ große Zugkraft nötig. Das gegenseitige axiale Verdrehen von Schlauchverbinder 1 und Schlauch 5 erleichtert das Ablösen der Innenwand des Schlauches 5 von der Außenseite des Anschlußstutzens 4.

Das gegenseitige axiale Verdrehen wird durch die Abflachungen 19 zusätzlich erleichtert. Weiterhin begünstigen die Abflachungen 1 9 ein sanftes Belüften eines eventuellen Restvakuums.

Sobald das Schlauchende 5a die Nuten 9 erreicht, kann - wie durch Pfeile Pf 3 gekennzeichnet - das bislang unter Unterdruck stehende Schlauchlumen 14 belüftet werden und zwar in einem Maße, daß ein allmählicher Druckausgleich eingeleitet wird und somit ein sauberes, zugkraftarmes und flüssigkeitsrückstandsfreies Trennen des Schlauches 5 von der Kupplung 1 gewährleistet ist. Die allmähliche Belüftung wird zusätzlich noch durch die Abschrägung 8 am Stutzen 4 begünstigt.

Durch diese Maßnahme wird verhindert, daß eventuell vorhandene Rest-Körper-Flüssigkeit aus dem Lumen des Schlauchendes 5a herausfließt und daß das Restvakuum in den Schläuchen 3, 5 schlagartig aufgehoben wird.

Durch die einströmende Luft wird die Rest-Flüssigkeit in Richtung PF innerhalb des Lumens 14 zum Auffangbehälter befördert und der Unterdruck allmählich abgebaut. Entsprechendes gilt für das Lumen 15 des Stutzens 4 bzw. des Lumen 16 des patientennahen Schlauches 3. Auch hier wird durch das allmähliche Einströmen von Luft in Richtung des Pfeiles Pf 4 eine Befreiung von Rest-Flüssigkeit bei der Entkopplung im Bereich der Schlauchkupplung sichergestellt.

Der körpernahe Schlauch 3 kann in der konischen Bohrung 2 des Stutzens 10 eingeklebt sein. Zur Versteifung kann das Gehäuse 11 Rippen 17 und einen Ringwulst 18 aufweisen.

## Patentansprüche

1. Schlauchkupplung (1) zum lösbaren Verbinden eines von einer Saugvorrichtung kommenden Schlauches (5) mit einem unlösbar mit der Schlauchkupplung (1) verbundenen Drainageschlauch (3) und mit einem auf der patientenfernen Seite (PF) angeordneten Stutzen (4), der an einem Ende eine Abschrägung (8) aufweist und zusammen mit einem Außengehäuse (11) einen in patientenferner Richtung (PF) offenen Schlauchaufnahmeraum (13) bildet, dadurch gekennzeichnet, daß der Stutzen (4) und die Innenseite (12) des Außengehäuses (11) konisch gestaltet sind und der Stutzen (4) auf seiner Außenseite etwa ab der Mitte mindestens eine in patientenferner Richtung (PF) bis zum patientennahen Ende (PN) der Abschrägung (8) laufende Nut (9) aufweist.

2. Schlauchkupplung nach Anspruch 1, dadurch gekennzeichnet, daß der Stutzen (4) in seinem patientennahen Bereich (PN) mindestens eine seitliche Abflachung (19) aufweist.

3. Schlauchkupplung nach Anspruch 1, dadurch gekennzeichnet, daß der Stutzen (4) regelmäßig auf seinem Umfang verteilte Abflachungen (19) aufweist.

## Claims

1. A hose coupling (1) for detachably connecting a hose (5) extending from a suction device, with a drainage hose (3) permanently connected with said hose coupling (1) and with a hub (4) disposed on the proximal side (PF), one end of said hub having a bevel (8) and forming, together with an outer housing (11), a hose receiving chamber (13) open in the proximal direction (PF), characterized in that said hub (4) and the inner side (12) of said outer housing (11) are conical and said hub (4) is provided with at least one groove (9) extending on its outer surface in the proximal direction (PF) from about the center of the hub to the distal end (PN) of said bevel (8).

2. The hose coupling of claim 1, characterized in that said hub (4) has at least one lateral flattening (18) in its distal region (PN).

3. The hose coupling of claim 1, characterized in that said hub (4) has flattenings (18) regularly distributed about its circumference.

## Revendications

1. Raccord (1) de tuyaux pour raccorder, de manière dissociable, un tuyau (5) en provenance d'un système d'aspiration à un tuyau de drainage (3) relié, de manière indissociable, au raccord (1) de tuyaux, et comportant un embout (4) disposé du côté (PF) éloigné du patient, présentant à une extrémité un biseau (8) et formant conjointement avec une enveloppe extérieure (11), une cavité de réception de tuyau (13) ouverte dans la direction (PF) s'éloignant du patient, caractérisé en ce que l'embout (4) et la face intérieure (12) de l'enveloppe extérieure (11) sont de configuration conique, et l'embout (4) présente sur sa face extérieure, environ à partir du milieu, au moins une rainure (9) s'étendant dans la direction (PF) s'éloignant du patient, jusqu'à l'extrémité (PN) du biseau (8) proche du patient.

2. Raccord de tuyaux selon la revendication 1, caractérisé en ce que l'embout (4) présente, dans sa partie (PN) proche du patient, au moins un méplat latéral (19).

3. Raccord de tuyaux selon la revendication 1, caractérisé en ce que l'embout (4) présente des méplats (19) régulièrement répartis sur sa périphérie.
